# EUROPEAN PATENT APPLICATION

(11) **EP 2 253 641 A2**
(43) Date of publication of application: **24.11.2010**
(21) Application number: 09154002.1
(22) Date of filing: 01.05.2008
(51) Int. Cl.: C07J 53/00

(54) **A process for preparing drospirenone and intermediate thereof**

(30) Priority: 01.05.2007 US 927242 P; 28.11.2007 US 990861 P; 19.03.2008 US 70207 P
(62) Divisional of application: 08767495.8
(71) Applicant: Sicor, Inc., Irvine, CA 92618 (US)
(72) Inventor: Pontiroli, Alessandro, S Maria Della Versa (IT); Diulgheroff, Nicola, Torino (IT); Scarpitta, Francesca, 10015, Ivrea (TO) (IT); Arosio, Roberto, I-23862, Civante (LC) (IT); Pogialli, Andrea, I-21042, Caronno Pertusella (IT); Villa, Marco, I-20125, Milano (IT)
(74) Representative: Gallagher, Kirk James

(57) **Abstract**

The present invention encompasses processes for preparing drospirenone and intermediates thereof.

## Description

### CROSS-REFERENCE TO RELATED APPLICATIONS

This application claims the benefit of U.S. provisional application Serial Nos. 60/927,242, filed May 1, 2007; 60/990,861, filed November 28,2007; and 61/070,207, filed March 19, 2008, hereby incorporated by reference.

### FIELD OF THE INVENTION

The present invention encompasses processes for preparing drospirenone and intermediates thereof.

### BACKGROUND OF THE INVENT

Drospirenone, 6β,7β;15β,16β-dimethylcne-3-oxo-17α-pregn-4-ene-21,17-carbolactone or (6R,7R,8R,9S,10R,13S,14S,15S,6S,17S)-1,3',4',6,6a,7,8,9,10,11,12,13,14,15,15a,16-hexadecahydro-10,13-dimethylspiro-[17H-dicyclopropa-6,7:15,16]cyclopenta[a]phenanthrene-17,2'(5H)-furan]-3,5'(2H)-dione) having the following structure: 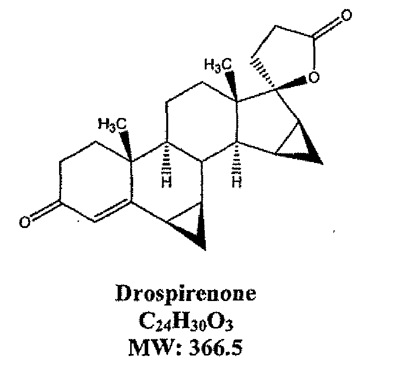 is a spironolactone analogue and a progestin with antimineralocorticoid property that acts to suppress gonadotropins. This pharmaceutical ingredient is commercially available as YASMTN®, which is administered for oral contraception as tablets containing 3 mg of drospirenone and 30 µg of ethinylestradiol.

Drospirenone is believed to be disclosed for the first time in U.S. Patent No. 4,129,564, where it is synthesized via the pathway described in Scheme 1 and each product produced after each reaction step is purified by column chromatography.

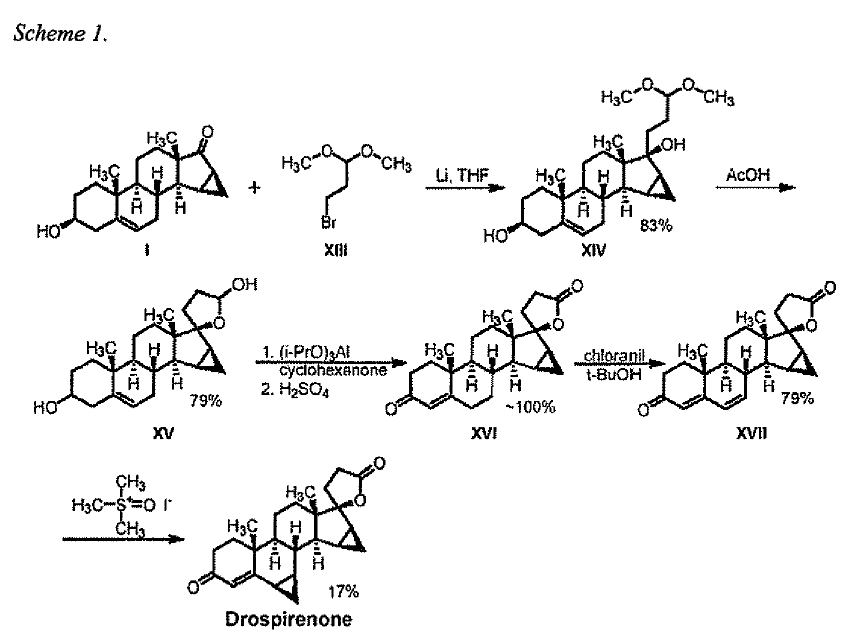

Each of U.S. Patent Nos. 4,435,327, 4,614,616 and 6,121,465 disclose a similar process for preparing drospirenone. The process is illustrated by Scheme 2. In this process, drospirenone is prepared from pivaloate of Formula IV by first transforming pivaloate of Formula IV into the key intermediate, androstanone of Formula VIII, followed by reacting androstanone of Formula VIII with propargyl alcohol and potassium methylate to provide the triol of Formula X. Then, reducing the triol of Formula X to intermediate of Formula XI by reacting the triol of Formula X with palladium on carbon (10% Pd/C catalyst) in a solvent mixture oftetrahydrofuran ("THF") /methanol and pyridine under hydrogenation. Lastly, oxidizing the intermediate of Formula XI to drospirenone, In addition, after each reaction step the products are purified, e.g. by column chromatography.

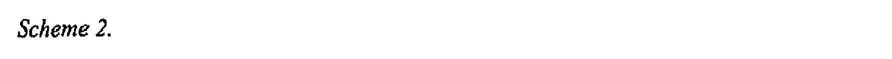

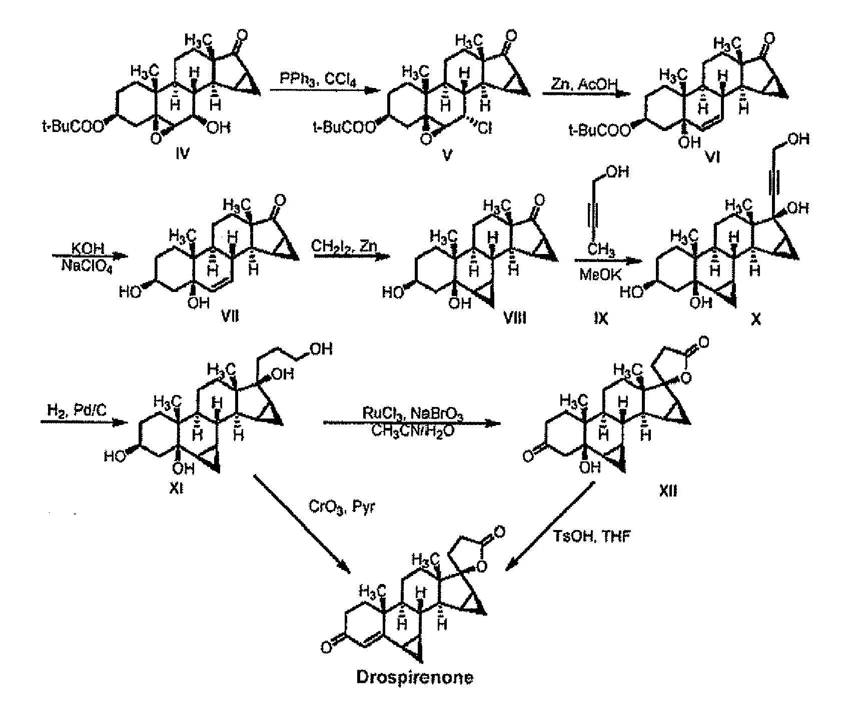

In U.S. Patent Nos. 4,435,327 and 4,614,616, the final oxidation is performed in the presence of chromium (VI) oxide. For example, reacting intermediate of Formula XI with chromium (VI) oxide in a mixture of pyridine and water, followed by recovering drospirenone in 62.5% yield after purifying and recrystallizing the product.

In U.S. Patent No. 6,121,465, the oxidation is carried out in the presence of ruthenium salts. For example, reacting intermediate of Formula XI with ruthenium trichloride and sodium bromate in a mixture of acetonitrile and water, thereby oxidizing intermediate of Formula X to 5-OH intermediate of Formula XII. Then reacting intermediate of Formula XII with *p*-toluenesulfonic acid in THF to provide drospirenone. Additional purification is done by chromatography.

U.S. Patent No. 4,904,462 describes a similar process for preparing drospirenone, in which the reduction of intermediate of Formula X to Formula XI is carried out by hydrogenating intermediate of Formula X with palladium on calcium carbonate (Pd/CaCO₃) in a solvent mixture of THF and isopropanol. Followed by oxidizing intermediate of Formula XI with pyridinium dichromate to provide drospirenone and isolating drospirenone by chromatography using semipreparative HPLC.

U.S. Patent No. 4,507,238 describes another approach to preparing drospirenone. For example, the patent describes reducing of the intermediate of Formula XI to Formula XII by hydrogenating intermediate of Formula XI with Raney Nickel in THF, and then oxidizing the intermediate of Formula XII with chromium trioxide and concentrating the reaction mixture in sulphuric acid to provide drospirenone.

For a variety of reasons, these processes are generally not suitable for industrial scale synthesis. For example, when highly toxic or carcinogenic reagents (e.g. chromium oxide or pyridine) are used in the preparation of drospirenone, more stringent safety measures are required for their usage and disposal. Another unfavourable aspect of these processes is when the reaction is carried out using metal catalysts, such as ruthenium salts and Raney Nickel because these metal catalysts are often expensive to obtain and not easy to handle. In addition, these processes require chromatographic purifications after almost each reaction step, which can be time consuming and expensive.

WO 2006/061309 and WO 2007/009821 provide an oxidation process that avoids the use of chromium derivatives. For example, the triol of Formula X is oxidized with sodium bicarbonate in water and calcium hypochlorite in the presence of TEMPO (2,2,6,6-tetramethylpiperidine-1-oxyl radical) (a reagent which is known to be expensive) to provide 5-OH intermediate of Formula XII and drospirenone is produced by then adding *p*-toluenesulfonic acid or dilute sulphuric acid to 5-OH intermediate of Formula XII.

The present invention provides an alternate process for preparing drospirenone. The present process focuses on the step-wise transformation of the compound of Formula VIII to drospirenone.

### SUMMARY OF THE INVENTION

In one embodiment, the present invention encompasses a process for preparing 17β-(3-hydroxy-1-propynyl)-6β,7β;15β,16β-dimethylene-5β-androstane-3β,5,17β-triol of Formula X: 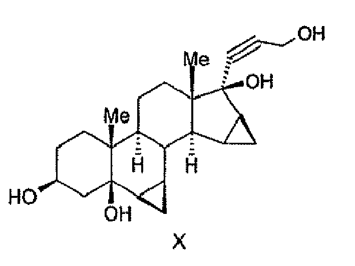 comprising reacting 3β,5-dihydroxy-6β,7β;15β,16β-dimethylene-5β-androst-17-one of Formula VIII ("androstanone"): 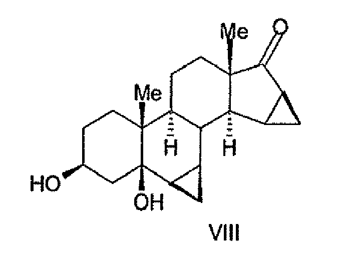 with about 1.8 to about 3 moles of propargyl alcohol per mole equivalent of androstanone and about 4 to about 6 moles of a base per mole equivalent of androstanone to provide a reaction mixture; and quenching the reaction mixture to obtain 17α-(3-hydroxy-l-propynyl)-6β,7β;15β,16β-dimethylene-5β-androstane-3[3,5,17β-triol, wherein the base is selected from the group consisting of: a base derived from a tertiary alcohol, an alkali metal hydride, an alkali metal amide, a C₄-C₈ alkyl lithium and mixtures thereof.

In another embodiment, the present invention encompasses a process for preparing drospirenone of the following formula: 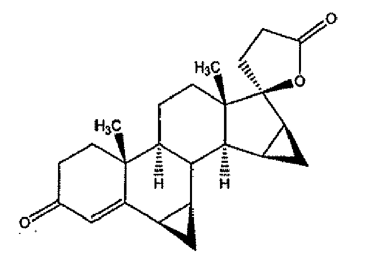 comprising preparing 17α-(3-hydroxy-1-propynyl)-6β,7β;15β,16β-dimethylene-5β-androstanc-3β,5,17β-triol of Formula X by the process of the present invention and converting it to drospirenone.

In another embodiment, the present invention encompasses a process for preparing 17α-(3-hydroxypropyl)-6β,7β;15β,16β-dimethylene-5β-androstan-3β,5,17β-triol of Formula XIa: 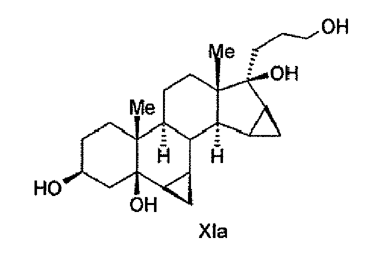 comprising reacting 17α-(3-hydroxy-1-propynyl)-6β,7β;15β, 16β-dimethylene-5β-androstane-3β,5,17β-triol of Formula X with at least one hydrogen source and a hydrogenation catalyst, wherein if the hydrogen source is hydrogen gas then the process further comprises a base that is not pyridine.

In another embodiment, the present invention encompasses a process for preparing drospirenone comprising preparing 17α-(3-hydroxypropyl)-6β,7β;15β,16β-dimethylene-5β-androstan-3β,5,17β-triol of Formula XIa by the process of the present invention and converting it to drospirenone.

In another embodiment, the present invention encompasses a process for preparing drospirenone comprising (a) reacting 17α-(3-hydroxypropyl)-6β,7β; 15β, 16β-dimethylene-5β-androstan-3β,5,17β-triol of Formula XIa with an alkali metal permanganate to provide a reaction mixture containing an intermediate of Formula XII: 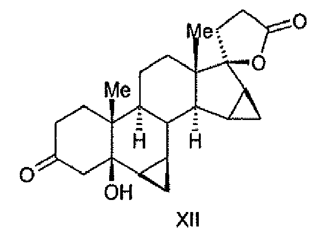 and (b) reacting the intermediate of Formula XII with an acid to provide drospirenone.

In another embodiment, the present invention encompasses a process for preparing drospirenone comprising:
a) reacting 3β,5-dihydroxy-6β,7β;15β,16β-dimethylene-5β-androst-17-one (Formula VIII) with about 1.8 to about 3 moles of propargyl alcohol per mole equivalent of 3β,5-dihydroxy-6β,7β;15β,16β-dimethylene-5β-androst-17-one and about 4 to about 6 moles of a base per mole equivalent of 3β,5-dihydroxy-6β,7β;15β,16β-dimethylene-5β-androst-17-one to provide a reaction mixture, wherein the base is selected from the group consisting of: a base derived from a tertiary alcohol, an alkali metal hydride, an alkali metal amide, a C₄-C₈ alkyl lithium and mixtures thereof;
b) quenching the reaction mixture to provide 17α-(3-hydroxy-1-propynyl)-6β,7β;15β,16β-dimethylene-5β-androstane-3β,5,17β-triol of Formula X;
c) reacting 17α-(3-hydroxy-1-propynyl)-6β,7β;15β,16β-dimethylene-5β-androstane-3β,5,17β-triol of Formula X with at least one hydrogen source selected from the group consisting of: hydrogen gas, sodium hypophosphite, ammonium formate, benzyl alcohol, allyl alcohol, cyclohexene, N-benzylaniline, formic acid, triethylammonium formate and mixtures thereof, and a hydrogenation catalyst, providing 17α-(3-hydroxypropyl)-6β,7β;15β,16β-dimethylene-5β-androstan-3β,5,17β-triol of Formula XIa, wherein if the hydrogen source is hydrogen gas then the process further comprises a base that is not pyridine;
d) reacting 17α-(3-hydroxypropyl)-6β,7β; 15β, 16-dimcthylene-5β-androstan-3β,5,17β-triol of Formula XIa with an alkali metal permanganate to provide a reaction mixture containing an intermediate of Formula XII; and
e) reacting the intermediate of Formula XII with an acid to provide drospirenone.

### DETAILED DESCRIPTION OF THE INVENTION

The present invention relates to a process for preparing drospirenone that uses inexpensive and non-toxic reagents, and provides a high purity product without the need to use chromatographic purification methods when purifying the products and its intermediates. In particular, no purifications are needed after each intermediate step. Further, the bases used in this process are strong bases in contrast to the weak bases used in the prior art processes. The weak bases of the prior art have greater selectivity than the strong bases used herein, nevertheless, we are able to develop reaction conditions that allowed achievement of the desired product yield and selectivity despite using these non-selective bases. Further, the strong bases required reagents in lesser amounts as compared to the prior art reaction that used weak bases. Therefore, for these reasons we believe the process may be suitable for industrial scale synthesis.

The process can be illustrated by the following scheme:

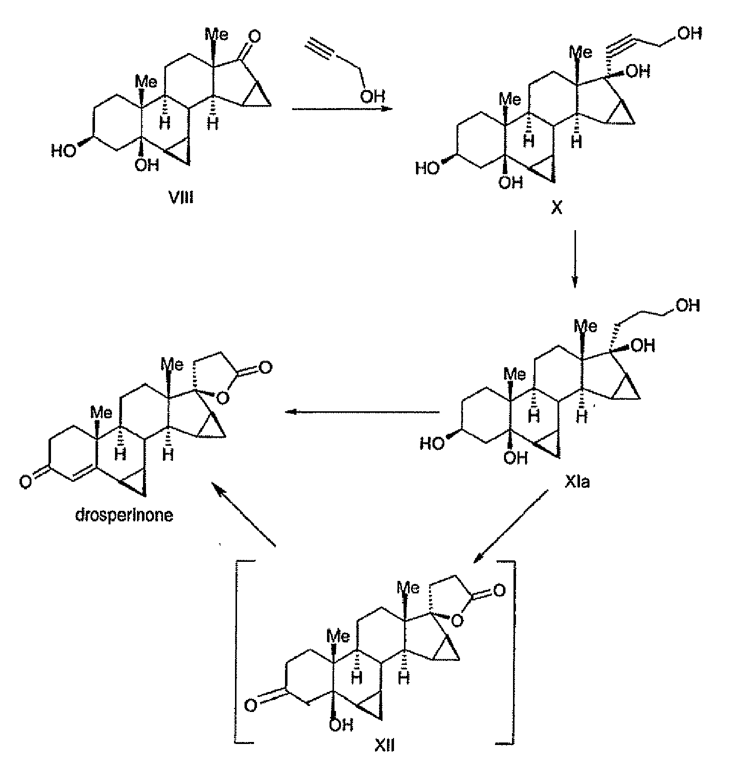

The first reaction step involves the formation of the compound of Formula X from androstanone of Formula VIII using significantly less amount of propargyl alcohol and base as compared to the amount used in U.S. Patent No. 4,435,327. For example, U.S. Patent No. 4,435,327 describes using about 11 moles of propargyl alcohol per mole equivalent of androstanone and about 14 moles of potassium methylate per mole equivalent of androstanone when preparing the compound of Formula X. The present process uses about 1.8 to about 3 moles of propargyl alcohol per mole equivalent of androstanone and about 4 to about 6 moles of an base per mole equivalent of Androstanone.

In one embodiment, the present invention encompasses a process for preparing 17α(3-hydroxy-1-propynyl)-6β,7β;15β,16β-dimethylene-5β-androstan-3β,5,17β-triol of Formula X: 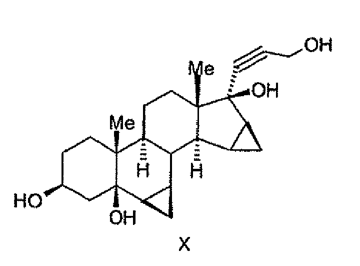 comprising reacting androstanone of Formula VIII: 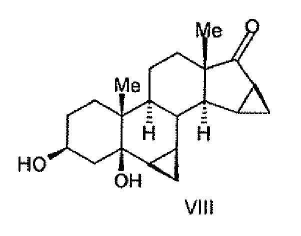 with about 1.8 to about 3 moles of propargyl alcohol per mole equivalent of androstanone and about 4 to about 6 moles of a base per mole equivalent of androstanone to provide a reaction mixture; and quenching the reaction mixture to obtain 17α-(3-hydroxy-1-propynyl)-6β,7β;15β,16β-dimethylene-5β-androstane-3β,5,17β-triol, wherein the base is selected from the group consisting of: a base derived from a tertiary alcohol, an alkali metal hydride, an alkali metal amide, a C₄-C₈ alkyl lithium and mixtures thereof.

Androstanone (3β, 5-dihydroxy-6β,7β;15β,16β-dimethylene-5β-androst-17-one) can be prepared using any known method in the art. For example, according to WO 2006/061309.

Preferably, prior to reacting with propargyl alcohol and the base, the androstanone (Formula VIII) is suspended in an aprotic organic solvent and then cooled. Preferably, the aprotic organic solvent is an ether. More preferably, the ether is a C₄₋₅ ether. Even more preferably, the C₄₋₅ ether is diethylether, tetrahydrofuran, dioxane, methyltetrahyrdrofuran ("MeTHF") or mixtures thereof. Most preferably, the aprotic organic solvent is THF. Preferably, the suspension is cooled to a temperature below 5°C. More preferably, it is cooled to a temperature of about -10°C to about 5°C. Most preferably, it is cooled to a temperature of about 0°C to about 5°C.

In most cases, about 1.8 to about 3 moles of propargyl alcohol per mole equivalent of androstanone is added to the suspension. Preferably, about 1.9 to about 2.2 moles of propargyl alcohol per mole equivalent of androstanone is added. Most often, about 4 to about 6 moles of the base per mole equivalent of androstanane is then added to the suspension. Preferably, about 4 to about 4.5 moles of the base per mole equivalent of androstanone is added.

As used herein, the term "a base derived from a tertiary alcohol" refers to a conjugate base of a tertiary alcohol. Preferably, a base derived from a tertiary alcohol is an alkali metal tert-alkoxide. More preferably, the alkali metal tert-alkoxide is sodium tert-butoxide or potassium tert-butaxide. Preferably, the alkali metal hydride is sodium hydride or potassium hydride. Preferably, the alkali metal amide is lithium diisopropyl amide ("LDA") or sodium amide. Preferably, the C₄-C₈ alkyl lithium is hexyllithium or butyllithium. The most preferred base is potassium tert-butoxide (also known as potassium tert-butylate).

Typically, the base used is a solid or it is dissolved in a solution. Preferably, the base used is dissolved in a solution. For example, when the base is potassium tert-butoxide, it can be dissolved in the same aprotic solvents as used in the reaction. Preferably, the base is dissolved in THF.

For best results, the reaction between androstanone, propargyl alcohol and the base is carried out at a temperature of about -5°C to about 0°C. Subsequently, a salt of compound of Formula X, such as a potassium salt, is formed.

The reaction mixture is subsequently quenched. The quenching, typically, transforms the salt of compound of Formula X to the compound of Formula X by reacting the salt of the compound of Formula X with a proton source. Preferably, the proton source is selected from the group consisting of an organic acid, an inorganic acid, water and mixtures thereof Examples of organic acids include, but are not limited to, acetic acid, methanesulphonic acid, *p*-toluenesulfonic acid, formic acid, tartaric acid, or citric acid. Examples of inorganic acids include, but are not limited to, sulphuric acid, phosphoric acid, hydrochloric acid, or hydrobromic acid.

The compound of Formula X can be recovered from the reaction mixture, for example, by adding acetic acid and water to the reaction mixture after quenching to form a two-phase system. Washing the reaction mixture further with THF and separating the two phases to provide the compound of Formula X.

The compound of Formula X can be used in the next reaction step without any purification. However, if purified, the compound of Formula X can be purified by crystallizing it from a mixture of THF and toluene. Preferably, the solvent ratio between THF and toluene is 1 to 3 by volume.

The compound of Formula X can subsequently be converted to drospirenone of the following Formula: 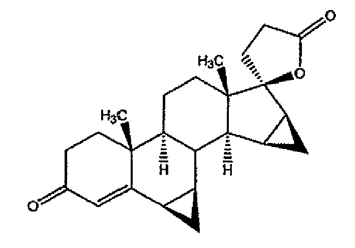 according to any method known to the skilled artisan, for example, according to WO 2006/061309, hereby incorporated by reference, or by the following process.

The compound of Formula X is first converted to a compound of Formula XI by reducing the triple bond of the propargyl moiety.

The compound of Formula XI can be formed as a mixture of two products, e.g. compounds of Formula XIa and Formula XIb: 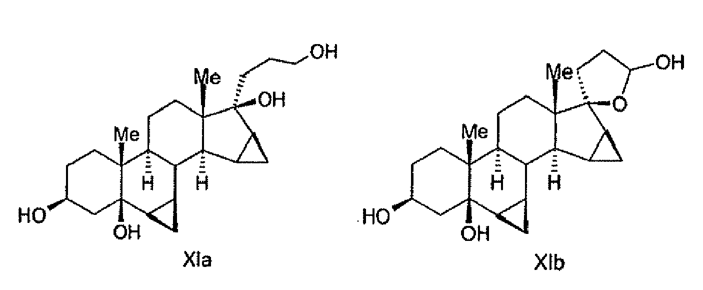 Typically, the compound of Formula XIa is present in the mixture at about 70% area percent as measured by HPLC and the compound of Formula XIb is present in the mixture at about 30% area percent as measured by HPLC.

One embodiment encompasses a process for preparing a compound of Formula XI comprising reacting the compound of Formula X with a hydrogenation catalyst and at least one hydrogen source, wherein if the hydrogen source is hydrogen gas, then the process further comprises a base that is not pyridine.

Generally, the reduction of the compound of Formula X to a compound of Formula XI can be carried out by using hydrogen gas (elemental hydrogen) as the hydrogen source or by using a hydrogen source selected from the group consisting of: sodium hypophosphite, ammonium formate, benzyl alcohol, allyl alcohol, cyclohexene, N-benzylaniline, formic acid, triethylammonium formate, and mixtures thereof along with a hydrogenation catalyst. This process can be especially attractive for large scale manufacture because when using hydrogen gas as the hydrogen source, a non-toxic base is used instead of pyridine, as most often, pyridine is used as a base under hydrogenation conditions and as a solvent in the prior art. In addition, the reduction can be performed with hydrogen sources other than hydrogen gas.

Preferably, about 1.5 to about 20 moles of the hydrogen source per mole equivalent of the compound of Formula X are added to the reaction mixture. More preferably, about 2.5 to about 10 moles of the hydrogen source per mole equivalent of the compound of Formula X are added to the reaction mixture.

Typically, the hydrogenation catalyst is selected from the group consisting of: palladium on calcium carbonate, palladium on charcoal, palladium black, palladium on barium sulphate, and mixtures thereof. Preferably, the hydrogenation catalyst is palladium on calcium carbonate or palladium on charcoal. Preferably, the hydrogenation catalyst is added in an amount of about 5% to about 10% by weight of the compound of Formula X.

When the hydrogen source is hydrogen gas, a base is also added to the reaction mixture as long as the base is not pyridine. The base added may be an organic base or an inorganic base. An example of the organic base include, but are not limited to, an aliphatic amine. Preferably, the organic base is triethylamine, diazabicycloundecene, diisopropylamine, or diisopropylethylamine. Examples of the inorganic base include, but are not limited to, sodium hydroxide, potassium carbonate, potassium bicarbonate or mixtures thereof. Preferably, the base is triethylamine or sodium hydroxide. The amount of base used is about 0.4 to 1 equivalent of base per mole of compound of Formula X, and preferably about 0.5 equivalents of base.

The solvent is chosen based on the hydrogenation catalyst and the hydrogen source added. When the hydrogenation catalyst is palladium on calcium carbonate and the hydrogen source is hydrogen gas, the solvent is preferably THF, ethyl acetate, methanol or mixture thereof. More preferably, the solvent used is THF.

For the most part, the hydrogen gas can be bubbled into a reaction mixture containing the compound of Formula X, the solvent, the hydrogenation catalyst and the base. Preferably, the hydrogen gas is kept at atmospheric pressure.

Another hydrogen source is sodium hypophosphite, ammonium format, benzyl alcohol, allyl alcohol, cyclohexene, N-benzylaniline, formic acid, triethylammonium formate, and mixtures thereof along with a hydrogenation catalyst. Preferably, the amount of ammonium formate added is at about 2 to about 20 moles of ammonium formate per mole equivalent of the compound of Formula X. More preferably, the amount of ammonium formate added is at about 2.5 to about 10 moles of ammonium formate per mole equivalent of the compound of Formula X.

When the hydrogen source is not hydrogen gas, the reaction can be performed without adding a base. In such cases, the reaction comprises reacting the compound of Formula X with a hydrogen source and a hydrogenation catalyst providing the intermediate of Formula XI. For example, when the hydrogen source is sodium hypophosphite or ammonium formate, they are typically added to a solution of compound of Formula X dissolved in solvent prior to adding the hydrogenation catalyst.

When not using hydrogen gas as the hydrogen source, the hydrogenation catalyst most often used is palladium on charcoal. Preferably, the amount of the hydrogenation catalyst added is at about 5% to about 20% by weight per gram of the compound of Formula X. More preferably, the amount of the hydrogenation catalyst added is at about 5% to about 8% by weight of the compound of Formula X.

Typically, when not using hydrogen gas as the hydrogen source, the solvent is selected from the group consisting of: alcohol, ester, ether and mixtures thereof. Optionally, water can be added to the reaction mixture. Preferably, the alcohol is a C₁-C₅ alcohol. More preferably, the C₁-C₅ alcohol is methanol. Preferably, the ester is a C₃-C₅ ester. More preferably, the C₃-C₅ ester is ethyl acetate. Preferably, the ether is a C₄-C₅ ether. More preferably, the ether is THF. The solvent may chosen based on the hydrogenation catalyst and the hydrogen source added. When the hydrogenation catalyst is palladium on charcoal and the hydrogen source is sodium hypophosphite or ammonium formate, the solvent is preferably a mixture of water and THF, ethyl acetate, methanol or mixture thereof. More preferably, the solvent used is a mixture of water and THF.

Optionally, a mixture of more than one hydrogen source can be added to the reaction mixture. In one example, the compound of Formula X is first combined with sodium hypophosphite, and after about 2 to about 3 hours, a hydrogenation catalyst and ammonium formate dissolved in an solvent are added.

Prior to reacting with the hydrogenation catalyst and the hydrogen source, the compound of Formula X is dissolved in an solvent selected from the group consisting of: tetrahydrofuran, mixtures of THF and water, ethyl acetate, methanol and mixtures thereof.

Typically, the reduction reaction is carried out at room temperature to about 50°C. Preferably, the reaction is carried out at a temperature of room temperature to about 40°C, More preferably, the reaction is carried out at a temperature of about 35°C to about 40°C. As used herein, the term "room temperature" refers to a temperature of about 20°C to about 25°C.

After reducing the compound of Formula X, a mixture containing compounds of Formula XIa and Formula XIb is obtained and can further be used in the subsequent reaction step for preparing drospirenone. Alternatively, the mixture can be converted to a compound of Formula XIa first prior to preparing drospirenone.

In most cases, the mixture containing compounds of Formula XIa and XIb is converted to a compound of Formula XIa first. Preferably, this conversion is achieved by mixing the reaction mixture obtained from the reduction step with a reducing agent, followed by quenching.

Suitable reducing agents include, but are not limited to, sodium borohydride, diisobutylaluminum hydride ("DIBALH"), lithium borohydride, disiamylbarane or mixtures thereof. Preferably, the reducing agent is sodium borohydride. Typically, the reducing agent is added to the reaction mixture in an amount of about 0.3 to about 1 mole per mole equivalent of the compound of Formula XIb. Preferably, it is added in an amount of about 0.5 to about 0.7 moles per mole equivalent of the compound of Formula XIb. More preferably, it is added in an amount of about 0.6 moles per mole equivalent of the compound of Formula XIb.

The quenching is carried out by adding a solvent such as water and ketone to the reaction mixture. Preferably, the ketone is acetone.

The compound of Formula XIa or its mixture with the compound of Formula XIb can then be recovered, for example, by filtering the reaction mixture and concentrating the filtrate.

The final step involves converting the compound of Formula XIa or its mixture with the compound of Formula XIb to drospirenone using any known methods in the art, for example, according to U.S. Patent No. 6,121,465, hereby incorporated by reference, or by the following process.

In another embodiment, the present invention encompasses a process for preparing drospirenone comprising (a) reacting 17α-(3-hydroxypropyl)-6β,7β;15β,16β-dimethylene-5β-androstan-3β,5,17β-triol of Formula XIa or its mixture with the compound of Formula XIb with an alkali metal permanganate to provide a reaction mixture containing an intermediate of Formula XII: 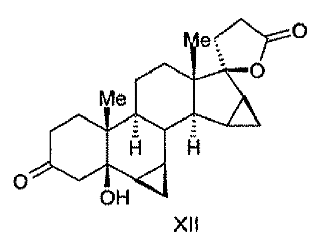 and (b) reacting the intermediate of Formula XII with an acid to produce drospirenone. Optionally, the reaction can be done without isolating the intermediate of Formula XII.

Usually, the reaction is carried out in the presence of a solvent. Typically, the solvent is selected from the group consisting of: ketone, water and mixtures thereof. Preferably, the ketone is a C₃-C₁₀ ketone. More preferably, the C₃-C₁₀ ketone is methyl ethyl ketone ("MEK"), acetone, diethylketone, diisopropylketone, methylisobutyl ketone, or mixtures thereof. Most preferably, the solvent is acetone or a mixture of acetone and water.

Preferably, the alkali metal permanganate is potassium permanganate or sodium permanganate. Most often, the alkali metal permanganate is added in an amount of about 2.5 to about 10 moles of potassium or sodium permanganate per mole of the compound of Formula XIa. Preferably, it is added in an amount of about 3 to about 5.5 moles of potassium or sodium permanganate per mole of the compound of Formula XIa. More preferably, it is added in an amount of about 4.5 to about 5.5 moles of potassium or sodium permanganate per mole of the compound of Formula XIa. Typically, potassium or sodium permanganate can be used as a solid or in an aqueous solution.

The reaction in step (a) is carried out at a temperature of about 25°C to about 50°C. Preferably, the reaction is carried out at a temperature of about 40°C to about 45°C. More preferably, the reaction is carried out at a temperature of about 40°C.

The compound of Formula XII can be recovered from the reaction mixture after step (a) or can react, *in-situ,* with an acid to provide drospirenone. The compound of Formula XII can be recovered, for example, by adding sodium metabisulfite to the mixture providing a two-phase system from which the compound of Formula XII is recovered after separating the two phases.

The acid used can be an organic acid or an inorganic acid. Examples of organic acids include, but are not limited to, formic acid, p-toluenesulfonic acid, methanesulfonic acid or mixtures thereof. Examples of inorganic acids include, but are not limited to, sulphuric acid, hydrochloric acid, hydrobromic acid, phosphoric acid or mixtures thereof. Preferably, the acid used is *p-*toluenesulfonic acid.

Drospirenone can be recovered, for example, by extracting and evaporating the reaction mixture.

Drospirenone can be purified by crystallizing it from a mixture of suitable organic solvents, *e.g.* ethyl acetate, toluene and n-heptane.

The present process would be attractive for large scale manufacture because the process avoids the use of highly toxic or carcinogenic reagents (e.g. chromium oxide or pyridine) when preparing drospirenone.

Having described the invention with reference to certain preferred embodiments, other embodiments will become apparent to one skilled in the art from consideration of the specification. The invention is further defined by reference to the following examples describing in detail the process for preparing drospirenone and intermediates thereof It will be apparent to those skilled in the art that many modifications, both to materials and methods, may be practiced without departing from the scope of the invention.

### EXAMPLES

### Example 1. Preparation of compound of Formula X

Androstanone of Formula VIII (90 g, 0.27 mol) was suspended in 235 ml of tetrahydrofuran at room temperature, the solution was cooled below 5°C and 30.6 g (0.54 mol, 2 eq) of propargyl alcohol was added dropwise. Then, a solution of 128.4 g (1.14 mol, 4.2 eq) of potassium tert-butylate dissolved in 990 ml of THF was added and the reaction was kept at 0°C for overnight. The product obtained was then isolated by the addition of 68.8 g of acetic acid and 90 ml of water. The reaction mixture was filtered on decalite pad and washed with 3 x 225 ml of THF. The filtered solution was then distilled under vacuum at 60°C to 270 ml residual volume. The two resulting phases were then separated. To the organic phase, 90 ml of water was added and the two phases were separated again. The combined organic phases were heated to reflux and 720 ml of toluene was then added in batches. After the initial 450 ml of toluene was added, the mixture was left stirring for 30 minutes then the remaining toluene was added. After 1 hour at 80°C the mixture was cooled to 5°C in 3 hours. After one hour, the obtained white solid was filtered and washed with 3 x 225 ml of toluene. As a result, 82 g of intermediate of Formula X was obtained (78% yield).

### Example 2: Example: preparation of compound of Formula X using potassium metilate

Androstanone of Formula VIII (24 g, 0.073 mol) was dissolved in 380 ml of tetrahydrofuran at room temperature, then the solution is cooled to 5°C and 6.67 g of potassium metilate (0.095 mol,1.17 eq) was added in about 30 minutes. At the end of the additions, a solution of 48.05 g (0.86 mol) of propargyl alcohol (1.3 eq) in 50 ml of THF was added drop-wise and the reaction was kept at 0°C for overnight. Only traces of the product were observed by HPLC.

### Example 3. Hydrogenation of intermediate of Formula X to mixture of intermediate of Formula XIa and XIb

Intermediate of Formula X (5 g, 13 mmol) was dissolved under nitrogen at room temperature in 75 ml of tetrahydrofuran and triethylamine (0.65 mg, 6.5 mmol) was added and stirred. Then 0.5 g of palladium on calcium carbonate, suspended in 25 ml of tetrahydrofuran was added at room temperature. The mixture was put under hydrogen atmosphere at atmospheric pressure for 16 hours. The mixture was then filtered, washed with tetrahydrofuran and then evaporated to dryness providing a mixture of intermediate of Formula XIa and XIb in 85% yield (in a ratio about 70/30 HPLC area percent).

### Example 4. Hydrogenation of intermediate of Formula X to XIa

Intermediate of Formula X (50 g, 130 mmol) was dissolved under nitrogen at room temperature in 750 ml of tetrahydrofuran and triethylamine (6.5 g, 65 mmol) was added and stirred. Then 5 g of palladium on calcium carbonate, suspended in 250 ml of tetrahydrofuran was added at room temperature. The mixture was put under hydrogen atmosphere at atmospheric pressure for 16 hours. Then sodium borohydride (2.9 g, 76 mmol) was dissolved in 18ml of water and added drop-wise to the reaction mixture. After 1 hour at room temperature the reaction was quenched by the addition of 22.4 ml of acetone. After one hour stirring at room temperature the reaction mixture was filtered on decalite pad and washed with 3 x 100 ml of THF. The filtered solution was concentrated under vacuum at 60°C until residual volume of 250 ml. Then, at 40°C, 750 ml of water was added drop-wise in about 2 hours and then the reaction mixture was cooled to about 5°C in 3 hours. After 1 hour a white solid was filtered on gooch P3 yielding 39 g of intermediate of Formula XIa (77.2% yield).

### Example 5. Hydrogenation of intermediate of Formula X to mixture of intermediate of Formula XIa and XIb

The intermediate of Formula X was dissolved in tetrahydrofuran/water (10 : 0.5 volumes) and 2.5 eq of sodium hypophosphite was added at room temperature with 0.2 volumes of sodium hydroxide 1M. The mixture was put to react with 5% w/w palladium on charcoal as hydrogenation catalyst at a temperature varying from 25°C to 40°C. The product was isolated by washings of the tetrahydrofuran solution with water and brine (95% yield).

### Example 6. Preparation of Drospirenone

Intermediate of Formula XIa (20 g) was put to oxidize in 1000 ml of acetone at 45°C. The mixture was then cooled to room temperature and 36.4 g of potassium permanganate (4.5 eq) was added in about 3.5 hours. The mixture was left stirring at room temperature for 24 hours. Then 300 ml of ethyl acetate and 30.94 g of sodium metabisulphite dissolved in 255 ml of water were added to the reaction mixture and the reaction mixture was vigorously stirred. Then another 295 ml of ethyl acetate was added together with 162 ml of sulphuric acid 1M, the two resulting phases were then separated and the aqueous phase was extracted once more with 200 ml of ethyl acetate and 50 ml of sulphuric acid 1M. The combined organic phases were washed with 3 x 200 ml of brine. The organic phase was then evaporated and slurried with diethyl ether providing a beige solid. The final product was purified by crystallization with a mixture of ethyl acetate/n-heptane.

### Example 7. Crystallization of Drospirenone (more detailed)

Drospirenone (9.5 g) was stirred in a flask with 62 ml of ethyl acetate. The mixture was heated to reflux (about 77°C) and 70 ml of n-heptane was then added dropwise in about 30 minutes. The reaction mixture was then cooled to 10°C in 3 hours. After keeping the reaction mixture overnight at 10°C, a solid was filtered and washed with 3x15 ml of n-heptane. As a result, 7.5 g of crystallized drospirenone was obtained (79% yield). Further aspects and features of the present invention are set out in the following numbered clauses.
1. A process for preparing 17α-(3-hydroxy-1-propinyl)-6β,7β;15β,16β-dimethylene-5β-androstane-3β,5,17β-triol of Formula X: 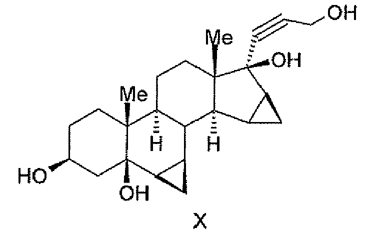 comprising reacting 3β,5-dihydroxy-6β,7β;15β,16β-dimethylene-5β-androst-17-one of Formula VIII: 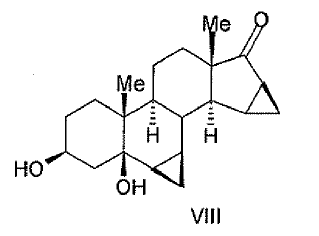 with about 1.8 to about 3 moles of propargyl alcohol per mole equivalent of 3β,5-dihydroxy-6β,7β;15β,16β-dimethylene-5β-androst-17-one and about 4 to about 6 moles of base per mole equivalent of 3β,5-dihydroxy-6β,7β;15β,16β-dimethylene-5β-androst-17-one to provide a reaction mixture; and quenching the reaction mixture to obtain 17α-(3-hydroxy-1-propinyl)-6β,7β;15v,16β-dimethylene-5β-androstane-3β,5,17β-triol, wherein the base is selected from the group consisting of: a base derived from a tertiary alcohol, an alkali metal hydride, an alkali metal amide, a C₄-C₈ alkyl lithium and mixtures thereof.
2. The process of clause 1, wherein the base is sodium tert-butoxide, potassium tert-butoxide, sodium hydride, potassium hydride, lithium diisopropyl amide, sodium amide, hexyllithium, butyllithium or mixtures thereof.
3. The process of clause 2, wherein the base is potassium tert-butoxide.
4. The process of any of clauses 1-3, wherein 3β,5-dihydroxy-6β,7β;15β,16β-dimethylene-5β-androst-17-one is suspended in an aprotic organic solvent prior to reacting with propargyl alcohol and the base.
5. The process of clause 4, wherein the aprotic organic solvent is a C₄₋₅ ether selected from the group consisting of diethylether, tetrahydrofuran, dioxane, methyltetrahyrdrofuran and mixtures thereof.
6. The process of any of clauses 1-5, wherein the quenching is carried out by reacting the reaction mixture with a proton source.
7. The process of clause 6, wherein the proton source is selected from the group consisting of: an organic acid, an inorganic acid, water and mixtures thereof.
8. A process for preparing drospirenone comprising preparing 17α-(3-hydroxy-1-propinyl)-6β,7β;15β,16β-dimethylene-5β-androstane-3β,5,17β-triol of Formula X according to the process of clause 1 and converting it to drospirenone.
9. A process for preparing 17α-(3-hydroxypropyl)-6β,7β;15β,16β-dimethylene-5β-androstan-3β,5,17β-triol of Formula XIa: 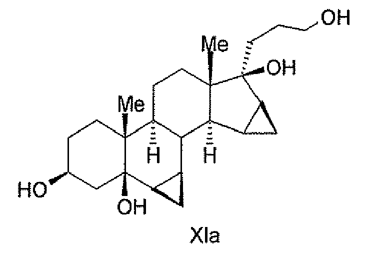 comprising (a) dissolving 17α-(3-hydroxy-1-propinyl)-6β,7β;15β,16β-dimethylene-5β-androstane-3β,5,17β-triol of Formula X in an organic solvent to provide a solution; and (b) reacting the solution with at least one hydrogen source and a hydrogenation catalyst, wherein if the hydrogen source is hydrogen gas then the process further comprises a base that is not pyridine.
10. The process of clause 9, wherein the organic solvent is selected from the group consisting of alcohol, ester, ether and mixtures thereof.
11. The process of clause 10, wherein the organic solvent is selected from the group consisting oftetrahydrofuran, mixtures of tetrahydrofuran and water, ethyl acetate, methanol and mixtures thereof.
12. The process of any of clauses 9-11, wherein the hydrogen source is selected from the group consisting of sodium hypophosphite, ammonium formate, benzyl alcohol, allyl alcohol, cyclohexene, N-benzylaniline, formic acid, triethylammonium formate and mixtures thereof.
13. The process of clause 12, wherein the hydrogen source is present in an amount of about 1.5 to about 20 moles per mole equivalent of 17α-(3-hydroxy-1-propinyl)-6β,7β;15β,16β-dimethylene-5β-androstane-3β,5,17β-triol of Formula X.
14. The process of any of clauses 9-13, wherein the hydrogenation catalyst is selected from the group consisting of: palladium on calcium carbonate, palladium on charcoal, palladium black, palladium on barium sulphate, and mixtures thereof.
15. The process of any of clauses 9-14, wherein the hydrogenation catalyst is present in an amount of about 5% to about 10% by weight per gram of 17α-(3-hydroxy-1-propinyl)-6β,7β;15β,16β-dimethylene-5β-androstane-3β,5,17β-triol of Formula X.
16. The process of any of clauses 9-15, wherein the base is triethylamine, diazabicycloundecene, diisopropylamine, diisopropylethylamine, sodium hydroxide, potassium carbonate, potassium bicarbonate or mixtures thereof.
17. The process of any of clauses 9-16, wherein the hydrogen source is hydrogen gas.
18. The process of clause 17, wherein the hydrogenation catalyst is palladium on calcium carbonate.
19. The process of clause 17, wherein the organic solvent is THF, ethyl acetate, methanol or mixture thereof.
20. The process of any of clauses 9-16, wherein the hydrogen source is sodium hypophosphite, ammonium formate, benzyl alcohol, allyl alcohol, cyclohexene, N-benzylaniline, formic acid, triethylammonium formate, and mixtures thereof.
21. The process of clause 20, wherein the amount of ammonium formate is at about 2 to about 20 moles of ammonium formate per mole equivalent of the compound of Formula X.
22. The process of any of clauses 20-21, wherein the hydrogenation catalyst is palladium on charcoal.
23. The process of any of clauses 20-22, wherein the amount of the hydrogenation catalyst added is at about 5% to about 20% by weight per gram of the compound of Formula X.
24. The process of any of clauses 20-23, wherein the organic solvent is selected from the group consisting of: alcohol, ester, ether and mixtures thereof.
25. The process of clause 24, wherein the alcohol is a C₁-C₅ alcohol, the ester is a C₃-C₅ ester, and the ether is a C₄-C₅ ether.
26. The process of clause 24, wherein the organic solvent is methanol, ethyl acetate, THF, or mixtures thereof.
27. A process for preparing drospirenone comprising (a) reacting 17α-(3-hydroxypropyl)-6β,7β;15β,16β-dimethylene-5β-androstan-3β,5,17β-triol of Formula XIa with an alkali metal permanganate to provide a reaction mixture containing an intermediate of Formula XII: 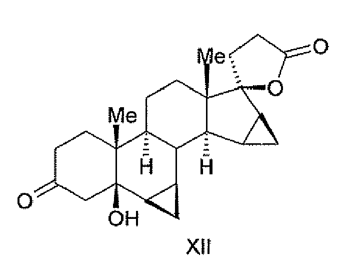 and (b) reacting the intermediate of Formula XII with an acid to provide drospirenone.
28. The process of clause 27, wherein the alkali metal permanganate is potassium permanganate or sodium permanganate.
29. The process of clause 27, wherein the alkali metal permanganate is present in an amount of about 2.5 to about 10 moles of potassium or sodium permanganate per mole of 17α-(3-hydroxy-1-propinyl)-6β,7β;15β,16β-dimethylene-5β-androstane-3β,5,17β-triol of Formula XIa.
30. The process of any of clauses 27-29, wherein the acid is formic acid, *p-*toluenesulfonic acid, methanesulfonic acid, sulphuric acid, hydrochloric acid, hydrobromic acid, phosphoric acid or mixtures thereof.
31. A process for preparing drospirenone comprising:
   a) reacting 3β,5-dihydroxy-6β,7β;15β,16β-dimethylene-5β-androst-17-one with about 1.8 to about 3 moles of propargyl alcohol per mole equivalent of 3β,5-dihydroxy-6β,7β;15β,16β-dimethylene-5β-androst-17-one and about 4 to about 6 moles of a base per mole equivalent of 3β, 5-dihydroxy-6β,7β;15β,16β-dimethylene-5β-androst-17-one to provide a reaction mixture, wherein the base is selected from the group consisting of: a base derived from a tertiary alcohol, an alkali metal hydride, an alkali metal amide, a C₄-C₈ alkyl lithium and mixtures thereof, according to any of clauses 1-5
   b) quenching the reaction mixture to provide 17α-(3-hydroxy-1-propinyl)-6β,7β;15β,16β-dimethylene-5β-androstane-3β,5,17β-triol of Formula X, according to any of clauses 6-7;
   c) reacting 17α-(3-hydroxy-1-propmyl)-6β,7β;15β,16β-dimethylene-5β-androstane-3β,5,17β-triol of Formula X with a hydrogen source selected from the group consisting of: hydrogen gas, sodium hypophosphite, ammonium formate, benzyl alcohol, allyl alcohol, cyclohexene, N-benzylaniline, formic acid, triethylammonium formate and mixtures thereof, and a hydrogenation catalyst providing 17α-(3-hydroxypropyl)-6β,7β;15β,16β-dimethylene-5β-androstan-3β,5,17β-triol of Formula XIa, wherein if the hydrogen source is hydrogen gas then the process further comprises a base that is not pyridine, according to any of clauses 9-26;
   d) reacting 17α-(3-hydroxypropyl)-6β,7β;15β,16β-dimethylene-5β-androstan-3β,5,17β-triol of Formula XIa with an alkali metal permanganate to provide a reaction mixture containing an intermediate of Formula XII, according to any of clauses 27-30; and
   e) reacting the intermediate of Formula XII with an acid to provide drospirenone.

## Claims

1. A process for preparing 17α-(3-hydroxy-1-propmyl)-6β,7β;15β,16β-dimethylene-5β-androstane-3β,5,17β-triol of Formula X: 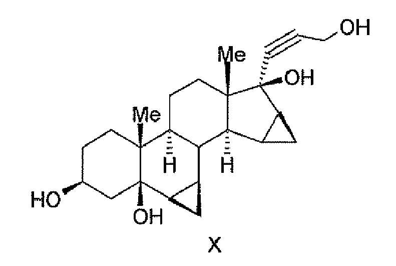 comprising reacting 3β,5-dihydroxy-6β,7β;15β,16β-dimethylene-5β-androst-17-one of Formula VIII: 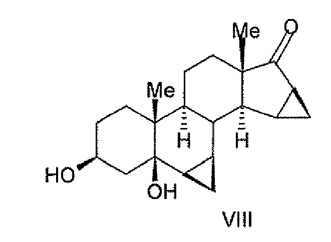 with about 1.8 to about 3 moles of propargyl alcohol per mole equivalent of 3β,5-dihydroxy-6β,7β;15β,16β-dimethylene-5β-androst-17-one and about 4 to about 6 moles of base per mole equivalent of 3β,5-dihydroxy-6β,7β;15β,16β-dimethylene-5β-androst-17-one to provide a reaction mixture; and quenching the reaction mixture to obtain 17α-(3-hydroxy-1-propinyl)-6β,7β;15β,16β-dimethylene-5β-androstane-3β,5,17β-triol, wherein the base is selected from the group consisting of: a base derived from a tertiary alcohol, an alkali metal hydride, an alkali metal amide, a C₄-C₈ alkyl lithium and mixtures thereof.

2. The process of claim 1, wherein the base is sodium tert-butoxide, potassium tert-butoxide, sodium hydride, potassium hydride, lithium diisopropyl amide, sodium amide, hexyllithium, butyllithium or mixtures thereof.

3. The process of claim 2, wherein the base is potassium tert-butoxide.

4. The process of any of claims 1-3, wherein 3β,5-dihydroxy-6β,7β;15β,16β-dimethylene-5β-androst-17-one is suspended in an aprotic organic solvent prior to reacting with propargyl alcohol and the base.

5. The process of claim 4, wherein the aprotic organic solvent is a C₄₋₅ ether selected from the group consisting of diethylether, tetrahydrofuran, dioxane, methyltetrahyrdroforan and mixtures thereof.

6. The process of any of claims 1-5, wherein the quenching is carried out by reacting the reaction mixture with a proton source.

7. The process of claim 6, wherein the proton source is selected from the group consisting of an organic acid, an inorganic acid, water and mixtures thereof.

8. A process for preparing drospirenone comprising preparing 17α-(3-hydroxy-1-propinyl)-6β,7β;15β,16β-dimethylene-5β-androstane-3β,5,17β-triol of Formula X according to the process of claim 1 and converting it to drospirenone.

9. A process for preparing drospirenone comprising:
a) reacting 3β,5-dihydroxy-6β,7β;15β,16β-dimethylene-5β-androst-17-one with about 1.8 to about 3 moles of propargyl alcohol per mole equivalent of 3β,5-dihydroxy-6β,7β;15β,16β-dimethylene-5β-androst-17-one and about 4 to about 6 moles of a base per mole equivalent of 3β, 5-dihydroxy-6β,7β;15β,16β-dimethylene-5β-androst-17-one to provide a reaction mixture, wherein the base is selected from the group consisting of: a base derived from a tertiary alcohol, an alkali metal hydride, an alkali metal amide, a C₄-C₈ alkyl lithium and mixtures thereof, according to any of claims 1-5
b) quenching the reaction mixture to provide 17α-(3-hydroxy-1-propinyl)-6β,7β;15β,16β-dimethylene-5β-androstane-3β,5,17β-triol of Formula X, according to any of claims 6-7;
c) reacting 17α-(3-hydroxy-1-propinyl)-6β,7β;15β,16β-dimethylene-5β-androstane-3β,5,17β-triol of Formula X with a hydrogen source selected from the group consisting of: hydrogen gas, sodium hypophosphite, ammonium formate, benzyl alcohol, allyl alcohol, cyclohexene, N-benzylaniline, formic acid, triethylammonium formate and mixtures thereof, and a hydrogenation catalyst providing 17α-(3-hydroxypropyl)-6β,7β;15β,16β-dimethylene-5β-androstan-3β,5,17β-triol of Formula XIa, wherein if the hydrogen source is hydrogen gas then the process further comprises a base that is not pyridine, according to any of claims 9-26;
d) reacting 17α-(3-hydroxypropyl)-6β,7β;15β,16β-dimethylene-5β-androstan-3β,5,17β-triol of Formula XIa with an alkali metal permanganate to provide a reaction mixture containing an intermediate of Formula XII, according to any of claims 27-30; and
e) reacting the intermediate of Formula XII with an acid to provide drospirenone.
